# EUROPEAN PATENT SPECIFICATION

(11) **EP 2 914 169 B1**
(45) Date of publication and mention of the grant of the patent: **01.12.2021**
(21) Application number: 13852295.8
(22) Date of filing: 31.10.2013
(51) Int. Cl.: A61B 5/02, H04B 7/24, A61N 1/05, A61B 5/00, A61B 5/145, H04B 5/00, A61N 5/06, A61N 7/00, A61B 5/0205, A61B 5/03, A61N 2/00, A61B 5/07, H01L 23/00, A61B 5/24, A61N 1/36, A61B 5/026

(54) **WIRELESS IMPLANTABLE SENSING DEVICES**
DRAHTLOSE IMPLANTIERBARE ERFASSUNGSVORRICHTUNGEN
DISPOSITIFS DE DÉTECTION IMPLANTABLES SANS FIL

(30) Priority: 31.10.2012 US 201261720827 P
(43) Date of publication of application: 09.09.2015
(73) Proprietor: The Board of Trustees of the Leland Stanford Junior University, Stanford, CA 94305-2038 (US)
(72) Inventor: POON, Ada, Shuk Yan, Palo Alto, CA 94306-1106 (US); HU, Bob, S., Palo Alto, CA 94306-1106 (US); JANG, Jihoon, Palo Alto, CA 94306-1106 (US); YAKOVLEV, Anatoly, Palo Alto, CA 94306-1106 (US); TANABE, Yuji, Palo Alto, CA 94306-1106 (US); YEH, Alex, Palo Alto, CA 94306-1106 (US); HSU, Stephanie, Palo Alto, CA 94306-1106 (US); MA, Andrew, Palo Alto, CA 94306-1106 (US)
(74) Representative: J A Kemp LLP
(86) International application number: PCT/US2013/067882
(87) International publication number: WO 2014/071079

(56) References cited:
- WO-A2-2009/008932
- US-A- 5 814 089
- US-A- 5 833 603
- US-A1- 2001 044 588
- US-A1- 2005 151 696
- US-A1- 2007 032 734
- US-A1- 2007 032 734
- US-A1- 2007 156 205
- US-A1- 2008 300 660
- US-A1- 2010 081 895
- US-A1- 2012 245 444
- ANATOLY YAKOVLEV ET AL: "Implantable biomedical devices: Wireless powering and communication", IEEE COMMUNICATIONS MAGAZINE, IEEE SERVICE CENTER, PISCATAWAY, US, vol. 50, no. 4, 1 April 2012 (2012-04-01), pages 152-159, XP011440723, ISSN: 0163-6804, DOI: 10.1109/MCOM.2012.6178849

## Description

### FIELD

This disclosure relates generally to implantable monitoring and sensing devices. More specifically, this disclosure relates to implantable, untethered, wireless monitoring and sensing devices for diagnostic purposes, and devices capable of control for therapeutic purposes.

### BACKGROUND

Cardiac arrhythmias affect more than 5 million people nationwide, and result in more than 1.2 million hospitalizations and 400,000 deaths each year in the United States. Atrial fibrillation (AF) and ventricular tachycardia (VT) account for most of the curable episodes if precise 3D mapping of the depolarization pattern is accessible. In the past five decades, various mapping systems have been proposed and developed. They can provide some but not all of the desired properties of an ideal mapping system.

The most common (AF) and the most lethal (VT) electrical disturbances of the heart are both caused by altered electrical conduction patterns. Therefore, the 3D mapping of the depolarization pattern has been an area of research for more than 5 decades. While conventional electrocardiogram (ECG) provides some ability to localize the pattern of depolarization, a more precise method would be highly desirable.

Methods like magnetocardiography (MCG) require highly specialized equipment and biomagnetic inversion problem is inherently ill-posed mathematically. MCG is a non-invasive mapping technique. But it requires the use of highly sensitive SQUID detector and suffers from sensitivity to noise due to the ill-posed inversion problem. Noninvasive techniques such as MCG have therefore been found unreliable for even investigative use.

Invasive intracardiac mapping is a laborious point-by-point mapping procedure that provides the only reliable analysis of the propagation electrical wavefront. Catheter-based activation and pacing in conjunction with surface electrocardiography and x-ray fluoroscopy is the most commonly used mapping technique in a clinical setting. However, the limitations of this technique are threefold. First, arrhythmia induction is often necessary for precise mapping. Patients who have structural heart disease (SHD) often have poor hemodynamic tolerance to the induced arrhythmia. Second, sequential recording is performed by a single or few electrode catheters maneuvered in the heart chamber. There is an implicit assumption that activations repeat in the same way from cycle to cycle at each site. This might not be a valid assumption in complex rhythms such as polymorphic arrhythmia. Thus, the procedure could last for several hours. Third, the mapping itself is limited to the reachable endocardial surface of the heart. Thus, local electrograms are not true representations of the depolarization pattern, especially in the thicker myocardium of the ventricles. Ideally, high-density maps of cardiac depolarization can be obtained without prolonged mapping.

Electrodes and stents have been implanted in human hearts for several decades. They have been deployed in the cardiac chambers as well as cardiac venous and arterial structures. Electrodes are currently millimeters in size and usually require a direct wired connection for operation. Stents measuring 50-100 microns in thickness are deployed routinely in the coronary arteries but lack of the ability to report back information from the local environment.

In the past few decades, newer mapping techniques have been proposed and developed. CARTO is the first 3D mapping system in electrophysiology (EP) testing. It utilizes a magnetic field sensor incorporated in the tip of the mapping catheter and an external magnetic field emitter located under the patient beneath the operating table. An electroanatomical map is generated when the mapping catheter is maneuvered in the heart chamber. The CARTO system, however, shares the same limitation of sequential recording and hence incurs long procedural time.

Non-contact multi-electrode mapping entails simultaneously recording of electrical activity at multiple sites. Other companies have developed a multi-electrode array with up to 64 electrodes in the shape of a balloon for endocardial mapping. Because the electrodes do not touch the endocardium, the mapping accuracy is limited.

In the UnEmap system developed by a group of researches in the University of Auckland, an epicardial electrode sock with over 100 electrodes is fitted over the heart. It is used extensively in experiments to better understand the underlying mechanisms of cardiac arrhythmias, but is seldom used in the clinical setting due to the invasiveness.

Optical mapping technique advances our understanding of cardiac electrophysiology in ways that have not been accomplished by other approaches. This technique uses a voltage-sensitive dye, invented by Nobel laureate Roger Tsien, to translate voltage changes into an optical signal, and provides better temporal and spatial resolution than other mapping techniques. Additionally, it allows simultaneous recording of membrane potential in the whole heart. Developed on experimental preparations using various species, these optical mapping techniques have recently been applied to the ex vivo human heart. These studies lead to the explanation of ventricular excitation and arrhythmias in terms of the hidden spatio-temporal patterns of propagation within the ventricular wall. However, the voltage-sensitive dyes are toxic. Therefore, optical mapping is not suitable for clinical use.

Table 1 summarizes the properties and limitations of the newer mapping techniques as compared with the conventional catheter-based technique and the invention disclosed herein.

**Table 1**

| | Catheter-based | CARTO | Endocardial multielectrode | Epicardial multielectrode | MCG | Optical mapping | This invention |
|---|---|---|---|---|---|---|---|
| Parallel recording | No | No | Yes | Yes | Yes | Yes | Yes |
| Minimally invasive | Yes | Yes | Yes | No | Yes | No | Yes |
| Non-toxic | Yes | Yes | Yes | Yes | Yes | No | Yes |
| Simple instruments | Yes | Yes | Yes | Yes | No | Yes | Yes |
| In contact with tissue | Yes | Yes | No | Yes | N/A | N/a | Yes |
| True intramyocardial recording | No | No | No | No | No | No | Yes |

US 2007/032734 A1 discloses a method of monitoring one or more physiological parameters for diagnosis of congestive heart failure within a patient. At least one sensing device is implanted in a cavity of the patient's cardiovascular system. The sensing device comprises an anchoring mechanism, at least one inductor coil, and at least one sensor operating to sense at least one of the one or more physiological parameters. A non-implantable readout device is provided that is not implanted in the patient. The readout device comprises at least one inductor coil having telemetric means for at least one of electromagnetic telecommunication and electromagnetic wireless powering of the sensing device through the at least one inductor coil of the sensing device.

US 5 814 089 A discloses an implantable system and method for delivering stimulus pulses and/or collecting data from a plurality of sites within a patient's body. The system has a main controller device with a power source, a stimulator/sensing devices at each of said sites, and circuitry for high frequency transmission of power from the main unit to each of the remote devices. Power is transferred by converting it into a high frequency at the controller unit, and periodically or on request transmitting it to the respective devices. The main controller unit and each respective device also preferably has one or more sensors for collecting data and processor circuitry for analyzing such data. Each remote device has a transmitter for transmitting collected data back to the main controller. The main controller has encoding circuitry for encoding a data component onto the high frequency carrier along with the power component. The controller unit and the respective devices are also equipped with circuitry for controlling power transmission on a need basis, i.e., when the remote device needs power and requests it.

US 2008/300660 A1 discloses an implantable, rechargeable medical system comprised of an implanted device, a power storage device connected to the implantable device, and a charging device operatively connected to the electrical storage device. The system may have long-range and short range wireless power harvesting capability.

US 5 833 603 A discloses a biosensing transponder for implantation in a human. A biosensor senses one or more physical properties related to the organism after the device has been implanted. A transponder wirelessly transmits data corresponding to the sensed parameter value to a remote reader. The transponder includes an energy coupler for wirelessly energizing the device with a remote energy source, and a control circuit for controlling and accessing the biosensor and for storing identifying data. The energy coupler can be an inductive circuit for coupling electromagnetic energy.

ANATOLY YAKOVLEV ET AL: "Implantable biomedical devices: Wireless powering and communication", IEEE COMMUNICATIONS MAGAZINE, IEEE SERVICE CENTER, PISCATAWAY, US, vol. 50, no. 4, 1 April 2012 (2012-04-01), pages 152-159, P011440723, ISSN: 0163-6804, DOI: 10.1109/MCOM.2012.6178849 discloses an active implantable device architecture and the associated components, and shows how a power link can be improved by utilizing focused powering via transmitter optimization, and provides a method for a high data rate and low power data transfer to and from the device.

### SUMMARY OF THE DISCLOSURE

The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
Figs. 1a-1c illustrate one embodiment of an implantable wireless sensing system.
Fig. 2 is a schematic drawing of a system comprising a sensing device and an external device.
Figs. 3a-3c provide additional views of the 3D structure of the sensing device of Fig. 1c.
Fig. 4 shows another embodiment of a sensing device.

### DETAILED DESCRIPTION

This disclosure describes methods and apparatus for replacing large "dumb" and tethered electrode sensing devices with multiple implantable or injectable "smart" untethered wireless-powered sensing devices. These untethered sensors can include electronics comprising integrated circuit (IC) chips configured to sense body parameters (e.g.,an electrogram),a wireless interface to transmit the sensed body parameters to an external device or detector, and a unique identification to locate each sensor. The systems described herein can include control over the types of body parameters to monitor, the duration of monitoring, and the number of locations within the body to monitor simultaneously.

This disclosure provides a novel mapping system comprising a plurality of implantable, wirelessly powered sensing and control devices configured to create a high-density map in real time or on demand of sensed body parameters (e.g.,cardiac depolarization) using simple and minimally invasive procedures and sensing devices without the need for prolonged mapping time.

An array of untethered, wirelessly-powered, small, and individually addressable electrode sensing devices can be implanted or injected into the body, such as into the circulatory system or into an organ, muscle, skin or body cavity of the patient. In a cardiac application, these sensing devices can be configured to detect the local depolarization patterns which can then be simultaneously interrogated by an external detector. The external detector can be adapted to demultiplex signals from these sensing devices at different locations, and reconstruct the depolarization map in real time. This mapping system can revolutionize the way of measuring bodily parameters such intracardiac electrical activities, assist cardiologists to ablate complicated arrhythmias, and reduce the procedural time of electrophysiology (EP) testing. In addition, it can provide medical researchers a flexible tool to better understand the electrical signal propagation and test out new hypothesis of the initiation of arrhythmias in cardiac tissue.

Wireless sensing and/or therapy devices disclosed herein can integrate the various circuit components of a wireless sensing device (e.g., wireless powering circuits, telemetry circuits, and the electrode sensors) into a single sensor IC chip. The sensing devices disclosed herein can include optimized designs for the implanted antenna, electrode configurations, 3D packaging of the entire probe, and the external detector. It is a goal of the systems described herein to minimize the effect of biological reaction to the presence of implanted wireless sensing devices.

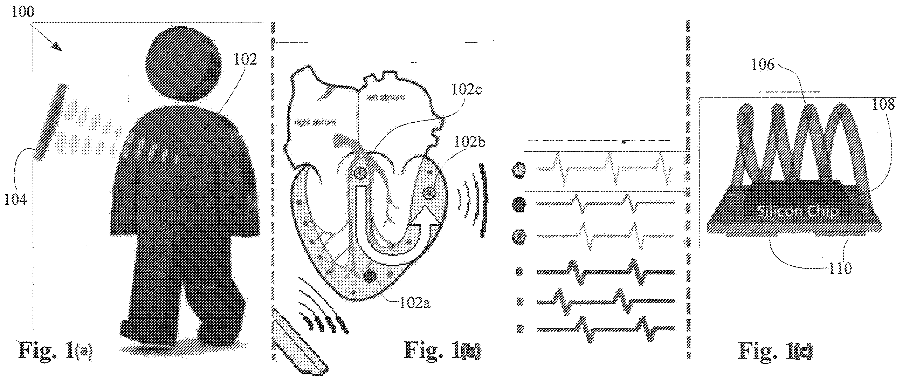

Figs. 1a-1c illustrate one embodiment of a wireless sensing system 100. Referring to Fig. 1c, the system 100 can include, for example, at least one implantable sensing device 102 configured to receive wireless power, and an external wireless power source and detector 104 configured to transmit wireless power and to transmit and/or receive communications. The wireless power source can include, for example, a transmitter coil connected to a power source. In some embodiments, the wireless power source can be separate from the detector, and in other embodiments they can be integrated into the same device. Any number of sensing devices can be implanted in the human body, depending on the bodily parameters to be sensed. For example, several sensing devices can be implanted on or within the heart of a patient to map local depolarization patterns of the heart.

As will be described in more detail below, the sensing devices are untethered, wirelessly powered sensing devices configured to measure a resistance of blood inside coronary stents and wirelessly communicate the sensed information to an external device. In some embodiments, the sensing devices can be configured to additionally sense one or more of the following: parameters of the body, such as ECG, EKG, EEG, resistance, or impedance, pressure parameters of the body such as the pressure of fluids within a lumen or an organ, temperature of the body or of bodily fluids, or optical parameters of the body, glucose content, or other chemical, biological, or particular molecular content in the blood or other organs. In some cases, certain composition of material can be sensed, such as presence of blood, puss, or bacterial infection. This can be achieved by including appropriate sensors on the sensing devices, such as electrodes, pressure sensors, temperature sensors, optical sensors, etc.

In one embodiment, a plurality of implanted sensing devices forms a network of sensing and stimulating devices that can operate in coordinated manner to close the loop for action with respect to measured quantities. For instance, in one embodiment, a device could measure cardiac output such as blood flow out of heart and another device could stimulate the heart to regulate the cardiac output.

Fig. 1b shows multiple sensing devices 102a, 102b, and 102c implanted at different locations in or on the heart. In some embodiments, the sensing devices can be implanted or injected directly into the myocardium of the heart, or alternatively, can be affixed to the epicardium or endocardium of the heart. Fig. 1b also shows various electrogram readings measured by the sensing devices. Although Fig. 1b shows the sensing devices being implanted in the heart, it should be understood that in other embodiments, the sensing devices can be implanted in other parts of the body.

In some embodiments, when the sensing devices are implanted within the heart of a patient, as shown in Fig. 1b, each sensing device can measure an electrical parameter such as the local electrogram. The external device or detector of the system can be configured to interrogate the measurement from each sensing device and deduce the propagation of excitation wavefront versus time. In some embodiments, the complete interrogation period for all sensing devices can be short enough compared to the cardiac signals in order to achieve high temporal resolution. Multiple sensing devices can be deployed within the body to simultaneously measure electrograms or other bodily parameters. Due to miniature form factor of the sensors, the numerous implanted devices can also achieve high spatial resolution.

Fig. 1c illustrates one embodiment of an implantable sensing device 102. The sensing device 102 can be delivered via injection, for example, and comprises an antenna 106, an integrated-circuit (IC) chip 108, and at least one sensor 110. In some embodiments, the antenna 106 can comprise a coil connected to the IC chip 108 to form a resonant or non-resonant system. The antenna 106 of the sensing device 102 can be inductively coupled to the wireless power source and detector 104 of Fig. 1a to receive wireless power and communications, and also to transmit communications to the detector. In some embodiments, the device size can be 1 x 1 x 1 mm, if sufficient power can be transferred to the device. In other embodiments, the device can be elongated in one dimension such that its diameter is small enough to fit in the needle, but has a larger antenna cross section for higher power harvesting capability. Depending on the operating environment, delivery method, and power consumption, a device could be as little as 100 µm and up to several centimeters in one or more dimensions.

In some embodiments, the sensor 102 can comprise an electrode. The sensor can be any type of sensor configured to measure a resistance of blood inside a coronary stent. In some embodiments, each sensing device can include a unique identification (ID) so as to identify the individual sensors. The unique ID can be used by the IC chip 108, or by an external detector (such as external detector 104) to identify the measurements taken by that individual sensor or induce localized stimulation to a particular region of the organ.

The IC chip 108 can be configured to provide signal processing directly on the sensing device. In some situations, it can be cheaper and more efficient to process the measured data on the sensing device itself, and transmit the processed data to the external device. Processing locally can be more efficient if the processing can be done with fairly simple analog or digital circuitry. The IC chip includes an energy harvesting and power management block. The IC chip may further include a matching network, a transceiver for telemetry with external reader, a sensor interface with signal conditioning circuitry, a signal conditioning and pulse generation block for stimulation, data conversion circuits, auxiliary circuit blocks, such as power-on-reset circuits, and a controller. Integrating all the electronics described into a single IC chip leads to having fewer discrete components and therefore allows the sensing devices to be miniaturized to the sizes described herein.

The controller manages the localized device operation, processes commands from the external reader, and packetizes and sends out sensed data back to the external reader. The controller can also contain digital or analog signal processing blocks which can analyze and process sensed data and can adjust the device's course of action or performance based on the processed information. For instance, if the controller determines that its analog-to-digital converter resolution is too low, it can increase its effective resolution or adjust gain of signal conditioning circuit to improve the performance, without engagement of the external reader. One other example for adjustment of performance is to automatically tune the cutoff frequencies of the filters in the sensor signal conditioning block in order to pass only the desired frequency components and filter out the interfering signals and noise. This may be done by the IC chip autonomously if all the necessary signal processing components are integrated on chip. Alternatively, the device can send raw data to the external reader. The reader can process the data and adjust the necessary parameters of the IC chip based on the information it receives by sending configuration commands to the IC chip.

Electronics and mechanical systems, empowered by modern CMOS, MEMS, and nanofabrication technologies, have been miniaturized faster than electro-chemical energy storage. As a result, embedded batteries typically dominate the size and weight of implanted medical devices. To combat this, the sensing devices of the present disclosure are powered externally by the external power source and detector via transcutaneous wireless power transfer. The external detector 102 can include a transmit coil that can be coupled to a receive coil on each sensing device. The receive coil can be, for example, the antenna 106 illustrated in Fig. 1c.

The transmit coil of the external detector and the receive coil of the sensing device can form a coupled circuit, whereby current flowing in the transmit coil can create a magnetic field which induces current to flow in the receive coil of the one or more sensing devices. This induced current can then be used to power the sensing devices. The sensing devices are configured to operate and measure bodily parameters only when they are in the presence of a magnetic field formed by the external device. Thus, the sensing device 102 can be miniaturized by not including a battery or energy source. When the antenna of the sensing device is in the presence of the magnetic field formed by the external device, the sensing device can turn on or "wake up" and collect or information relating to body parameters. The sensing device can also communicate this sensed information externally while receiving wireless power.

Traditional wireless power transfer across human tissue operates in the near-field, where the transmit and receive coils include inductively coupled coils. Recently proposed systems for mid-range power transfer over air and through tissue also occur in the near-field; high efficiency can be obtained by tuning identical resonators to operate in the strongly coupled regime. Power transfer to medical implants, however, typically operates in the weakly coupled regime due to the asymmetry between a large external transmit coil and the small receive coil on the implant. In this configuration, it has been shown that optimal power transfer occurs in the mid-field where energy is exchanged through a combination of inductive and radiative modes.

Typical implantable devices rely on inductive coupling to harvest power and communicate with the external transmitter. This often implies large coil antennas for both the external transmitter and the implant device antenna. One of the key disadvantages of the inductive coupling is that these antennas cannot be significantly miniaturized because they become very inefficient. Increasing the frequency of operation increases the tissue absorption and tissue heating, however, it also increases antenna efficiency for very small antennas. Therefore, there is an optimal frequency at which enough power can be delivered to the small antennas, while limiting tissue heating to safe levels. The optimal frequency of operation for the implantable sensors disclosed herein is from 100 MHz up to 5 GHz, depending on implantable device design, operating environment, required power and voltage, and device size.

Operating the implantable sensing devices of this disclosure for mid-range power transfer over high operating frequencies allows for miniaturization of the sensing devices that would not be possible in a system that uses inductive coupling. For example, sensing devices of the present disclosure can be miniaturized to have a total volume on the order of 1mm x 1mm x 1mm. Another benefit of higher frequency of operation is the higher available bandwidth that can be used to achieve higher data rates for communication. Also, higher frequency of operation desensitizes power transfer efficiency to alignment and orientation between external and implant device antennas.

Wirelessly powering the sensing devices of the present disclosure eliminates the need for leads extending through the skin, which simplifies the implantation procedure and reduces the risk of infection. The sensing devices described herein are therefore less invasive, and safer in the long term for a patient. This is especially important in applications such as brain or cardiac monitoring, where the risk of infection is so great. This also enables longer-lasting implantable devices by eliminating the need for a patient to undergo another surgery to replace a battery.

The sensing devices of Figs. 1a-1c are configured for wireless communication of data, to communicate the measured body parameters outside of the body. This data can be communicated to the external device via a wireless chip incorporated into the IC chip 108, or alternatively, the data can be modulated onto the wireless power transfer signals between the transmission and receive coils of the external device and sensing devices, respectively. The wireless communication of data occurs only during wireless power transfer between the external device and the implanted sensing devices, so as to reduce power consumption of the device during normal operation.

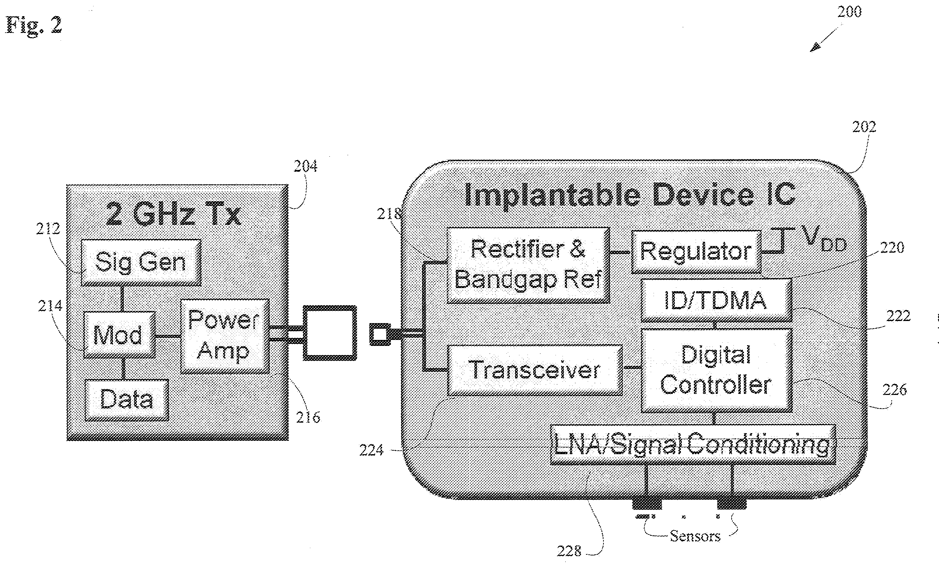

Fig. 2 is a schematic drawing of a system 200 comprising sensing device 202 and external device 204. The sensing device 202 and external device 204 can correspond to sensing device 102 and external device 104 of Figs. 1a-1c. The external device can wirelessly transfer power and data with a signal generator 212, a frequency modulator 214, and one or more power amplifiers 216. In Fig. 2, the individual components of the sensing device (e.g., rectifier and bandgap ref 218, regulator 220, ID/TDMA 222, transceiver 224, digital controller 226, LNA/signal conditioning module 228, etc) can be incorporated into the IC chip shown above in Fig. 1c.

Referring to Fig. 2, as recorded signals from the sensors (e.g., electrodes) are corrupted by motion artifact, dc-offset due to the skin-electrode contact resistance, and the 60-Hz interference, the sensor frontend LNA/signal conditioning module 238 can process the measured extracellular signal to obtain a clean signal and detect the timing of local depolarization. All the handshaking protocols with the external device, for example, the multiple access protocol, and the coordination among various building blocks within the cardiac probe can be coordinated by the digital controller 226. The ID of individual probe can be stored in the ID block 222. The transceiver block 224 modulates the processed intracellular signals and sends it to the external device, and demodulates received signals, for example, commands, from the external device. The rectifier 218 and regulator blocks 220 convert the oscillating radio waves incident on the received antenna to dc power for the operation of the sensing device.

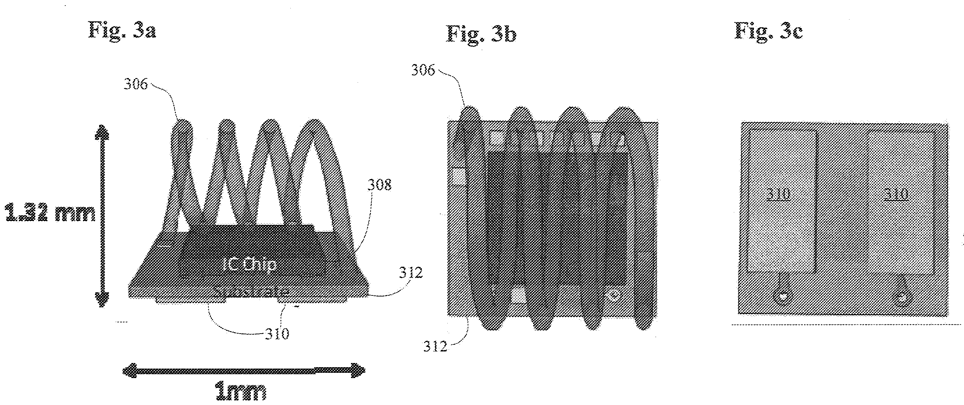

Figs. 3a-3c provide additional details on the 3D structure of one embodiment of the sensing devices (expanding on what is shown in Fig. 1c). Since it is desirable to have the entire implanted device be as small as possible, this embodiment adopts a 3D packaging approach. The implanted antenna 306 can be a multi-turn micro-coil, for example. The antenna can be wire-bonded to the pads on a supporting substrate 312. The IC chip 308 and sensors 310 can also be bonded to the substrate. Fig. 3b shows a top-down view of the sensing device, and Fig. 3c shows a bottom-up view of the sensing device, giving a better view of sensors 310. In some embodiments, the sensing devices of Figs. 3a-3c can be approximately 1mm wide, 1mm long, and 1.3mm tall. In another embodiment, the sensing device can be 1mm x 1mm x 1mm.In another possible embodiment, the device can be encapsulated in an optically transparent package that would enable optical methods of energy harvesting, stimulation, and sensing. Other types of transparency for packaging can be extended to include RF transparent materials, etc.

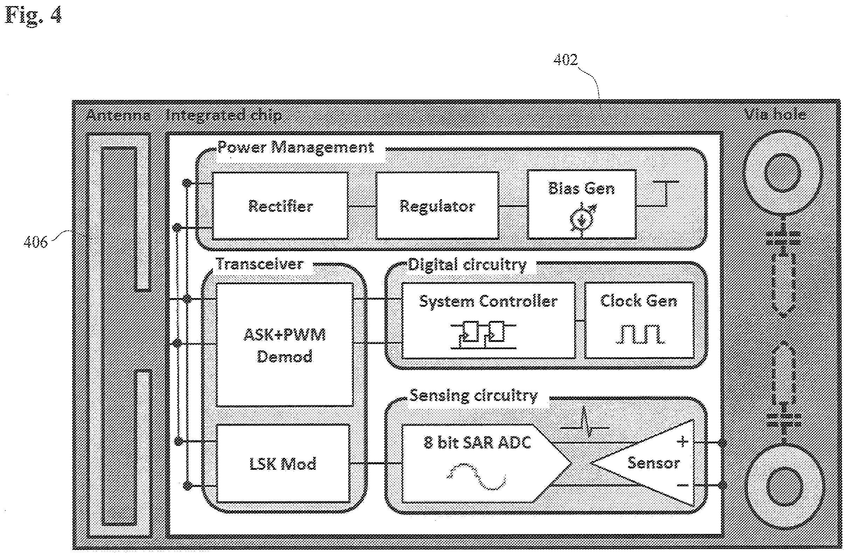

Fig. 4 shows another embodiment of a sensing device 402. In this embodiment, a planar loop antenna 406 can be used for the implantable device. The tradeoff between multi-turn antenna in the form of 3D coil versus a planar antenna with the same diameter, is that the 3D coil antenna has higher induced voltage, which can be advantageous to improve rectifier efficiency. However, it has additional losses associated with it and, therefore, can harvest less power as compared to a planar loop antenna. The illustrated components of the IC chip in Fig. 4 can correspond to the IC chip components described above in Fig. 2.

Compact, wirelessly powered, untethered sensing devices such as those described herein can be used in any number of medical applications. As described above, an array of sensing devices can be used to measure and map the electrical properties of the heart, and communicate that information wirelessly outside of the body to an external device. Uses within the heart are not limited to electrical properties, however, and the sensing devices can also be used to monitor blood flow, heart rate, core temperature, and more.

In another embodiment, a plurality of sensing devices outfitted with pressure sensors can be implanted within the venous system of a patient (e.g., within the pulmonary arteries) to measure the pressure of blood or the flow rate of blood at various points in the venous system.

According to the present invention a plurality of sensing devices outfitted with electrical sensors or electrodes can be disposed on or near coronary stents and are configured to measure a resistance of blood inside the coronary stents. In this way the sensing devices can monitor the degree of stenosis inside the stent and could be used to report when a stent is wearing down and due for replacement. Thus, the sensing devices of the present invention can be used to monitor the effectiveness and lifetime of other medical devices implanted in the body.

The sensing devices described herein can also be implanted on or within the brain to measure various parameters relating to electrical activity, blood flow, or pressure in the brain. For example, in some situations surgeons need info on the local perfusion of the brain, since swelling after surgery can compromise that part of the brain. Such sensing devices can be implanted in the brain to measure blood flow or other brain parameters as a way of monitoring the patient after surgery. The sensing devices described herein allow for measurement of brain activity directly without having leads that extend out through the skull, thereby reducing infection risk.

Until this point, the sensing devices have been described entirely as sensing or measurement devices. However, in some embodiments, the sensing devices can also include a therapy element, such as a stimulation electrode, configured to provide therapy to a patient. For example, the sensors 110 of Fig. 1c can comprise electrodes and can be configured to provide, for example, cardiac or deep brain stimulation. In other embodiments, the therapy element can be configured to provide magnetic, electrical, optical, ultrasound, or chemical stimulation to bodily tissue or fluids.

As for additional details pertinent to the present invention, materials and manufacturing techniques may be employed as within the level of those with skill in the relevant art. The same may hold true with respect to method-based aspects of the disclosure in terms of additional acts commonly or logically employed. Also, it is contemplated that any optional feature of the inventive variations described may be set forth and claimed independently, or in combination with any one or more of the features described herein. Likewise, reference to a singular item, includes the possibility that there are plural of the same items present. More specifically, as used herein and in the appended claims, the singular forms "a," "and," "said," and "the" include plural referents unless the context clearly dictates otherwise. As such, this statement is intended to serve as antecedent basis for use of such exclusive terminology as "solely," "only" and the like in connection with the recitation of claim elements, or use of a "negative" limitation. Unless defined otherwise herein, all technical and scientific terms used herein have the same meaning as commonly understood by one of ordinary skill in the art to which this invention belongs. The breadth of the present invention is not to be limited by the subject specification, but rather only by the plain meaning of the claim terms employed.

## Claims

1. A patient monitoring system (100, 200), comprising:
a plurality of implantable, untethered sensing devices (102, 102a, 102b, 102c, 202, 402) configured to be disposed on or near coronary stents implanted in a patient's body, each untethered sensing device comprising:
an antenna (106, 306, 406) configured to receive wireless power from a power source external to the patient;
at least one electrical sensor or electrode (110, 310) configured to measure a resistance of blood inside a said coronary stent; and
electronics (108, 308) coupled to the antenna and the at least one sensor or electrode and configured to communicate the measured blood resistance to a device (104, 204) external to the patient, wherein the electronics comprise an energy harvesting and power management block and a digital controller (226) and wherein the controller is configured to manage the localized device operation, process commands from the external device, and packetize and send out sensed data back to the external device; and
at least one said external device (104, 204) containing the power source and configured to provide wireless power to the antennae of the sensing devices at an operating frequency ranging from 100 MHz to 5 GHz and configured to receive the measured blood resistance from the sensing devices and enable monitoring of the effectiveness and lifetime of the coronary stents implanted in the patient's body;
wherein the sensing devices are configured to be powered only when receiving wireless power from the external device and to be turned on only when their antennae are in the presence of a magnetic field formed by said at least one external device.

2. The system of claim 1, wherein the system is configured to monitor the degree of stenosis inside the coronary stents based on the measured resistance of blood inside the coronary stents.

3. The system of claim 1, wherein each of the plurality of untethered sensing devices (102, 102a, 102b, 102c, 202, 402) is individually addressable.

4. The system of claim 1, wherein the antenna of each sensing device (102, 102a, 102b, 102c, 202, 402) is a planar loop antenna (406).

5. The system of claim 1, wherein the electronics of each sensing device (102, 102a, 102b, 102c, 202, 402) comprises a pulse generator block for stimulation.

6. The system of claim 1, wherein each of the plurality of untethered sensing devices (102, 102a, 102b, 102c, 202, 402) measures less than 1mm x 1mm x 1mm in size.

## Patentansprüche

1. Patientenüberwachungssystem (100, 200), umfassend:
eine Vielzahl von implantierbaren, ungebundenen Erfassungsvorrichtungen (102, 102a, 102b, 102c, 202, 402), die konfiguriert sind, um an oder in der Nähe von Koronarstents, die in den Körper eines Patienten implantiert sind, angeordnet zu sein, wobei jede ungebundene Erfassungsvorrichtung Folgendes umfasst:
eine Antenne (106, 306, 406), die konfiguriert ist, um drahtlose Energie von einer Energiequelle außerhalb des Patienten zu empfangen;
mindestens einen elektrischen Sensor oder mindestens eine Elektrode (110, 310), der/die konfiguriert ist, um den Blutwiderstand im Inneren des Koronarstents zu messen; und
eine Elektronik (108, 308), die mit der Antenne und dem mindestens einen Sensor oder der mindestens einen Elektrode gekoppelt und konfiguriert ist, um den gemessenen Blutwiderstand an eine Vorrichtung (104, 204) außerhalb des Patienten zu übermitteln, wobei die Elektronik einen Energiegewinnungs- und Leistungsverwaltungsblock und eine digitale Steuerung (226) umfasst und wobei die Steuerung konfiguriert ist, um den lokalisierten Vorrichtungsbetrieb zu verwalten, Befehle von der externen Vorrichtung zu verarbeiten und die erfassten Daten zu paketieren und zurück an die externe Vorrichtung zu senden; und
mindestens eine externe Vorrichtung (104, 204), die die Leistungsquelle enthält und konfiguriert ist, um die Antennen der Erfassungsvorrichtungen bei einer Betriebsfrequenz im Bereich von 100 MHz bis 5 GHz mit drahtloser Energie zu versorgen, und konfiguriert ist, um den gemessenen Blutwiderstand von den Erfassungsvorrichtungen zu empfangen und die Überwachung der Wirksamkeit und Lebensdauer der in den Körper des Patienten implantierten Koronarstents zu ermöglichen;
wobei die Erfassungsvorrichtungen konfiguriert sind, um nur dann betrieben zu werden, wenn sie drahtlose Energie von der externen Vorrichtung empfangen, und nur dann eingeschaltet zu werden, wenn sich ihre Antennen in der Gegenwart eines Magnetfeldes befinden, das von der mindestens einen externen Vorrichtung gebildet wird.

2. System nach Anspruch 1, wobei das System konfiguriert ist, um den Grad der Stenose innerhalb der Koronarstents beruhend auf dem gemessenen Blutwiderstand innerhalb der Koronarstents zu überwachen.

3. System nach Anspruch 1, wobei jede der Vielzahl von ungebundenen Erfassungsvorrichtungen (102, 102a, 102b, 102c, 202, 402) individuell adressierbar ist.

4. System nach Anspruch 1, wobei die Antenne jeder Erfassungsvorrichtung (102, 102a, 102b, 102c, 202, 402) eine planare Schleifenantenne (406) ist.

5. System nach Anspruch 1, wobei die Elektronik jeder Erfassungsvorrichtung (102, 102a, 102b, 102c, 202, 402) einen Impulsgeneratorblock zur Stimulation umfasst.

6. System nach Anspruch 1, wobei jede der Vielzahl von ungebundenen Erfassungsvorrichtungen (102, 102a, 102b, 102c, 202, 402) weniger als 1 mm x 1 mm x 1 mm groß ist.

## Revendications

1. Système de surveillance de patient (100, 200), comprenant :
une pluralité de dispositifs de détection implantables et autonomes (102, 102a, 102b, 102c, 202, 402) configurés pour être disposés sur ou à proximité de stents coronaires implantés dans le corps d'un patient, chaque dispositif de détection autonome comprenant :
une antenne (106, 306, 406) configurée pour recevoir une alimentation sans fil à partir d'une source d'alimentation externe au patient ;
au moins un capteur ou électrode électrique (110, 310) configuré pour mesurer une résistance du sang à l'intérieur d'un dit stent coronaire ; et
une électronique (108, 308) couplée à l'antenne et à l'au moins un capteur ou électrode et configurée pour communiquer la résistance sanguine mesurée à un dispositif (104, 204) externe au patient, dans lequel l'électronique comprend un bloc de récupération d'énergie et de gestion de l'alimentation et un contrôleur numérique (226) et dans lequel le contrôleur est configuré pour gérer le fonctionnement du dispositif localisé, traiter les commandes du dispositif externe, et mettre en paquets et envoyer les données détectées au dispositif externe ; et
au moins un dit dispositif externe (104, 204) contenant la source d'alimentation et configuré pour fournir une alimentation sans fil aux antennes des dispositifs de détection à une fréquence de fonctionnement allant de 100 MHz à 5 GHz et configuré pour recevoir la résistance sanguine mesurée à partir des dispositifs de détection et permettre de surveiller l'efficacité et la durée de vie des stents coronaires implantés dans le corps du patient ;
dans lequel les dispositifs de détection sont configurés pour être alimentés uniquement lorsqu'ils reçoivent une alimentation sans fil à partir du dispositif externe et pour être allumés uniquement lorsque leurs antennes sont en présence d'un champ magnétique formé par ledit au moins un dispositif externe.

2. Système selon la revendication 1, dans lequel le système est configuré pour surveiller le degré de sténose à l'intérieur des stents coronaires sur la base de la résistance mesurée du sang à l'intérieur des stents coronaires.

3. Système selon la revendication 1, dans lequel chacun de la pluralité de dispositifs de détection autonomes (102, 102a, 102b, 102c, 202, 402) est adressable individuellement.

4. Système selon la revendication 1, dans lequel l'antenne de chaque dispositif de détection (102, 102a, 102b, 102c, 202, 402) est une antenne à boucle planaire (406).

5. Système selon la revendication 1, dans lequel l'électronique de chaque dispositif de détection (102, 102a, 102b, 102c, 202, 402) comprend un bloc générateur d'impulsions pour la stimulation.

6. Système selon la revendication 1, dans lequel chacun de la pluralité de dispositifs de détection autonomes (102, 102a, 102b, 102c, 202, 402) mesure moins de 1 mm × 1 mm × 1 mm.
